Europäisches Patentamt

European Patent Office

Office européen des brevets

(11) Publication number: **0 375 681 B1**

# EUROPEAN PATENT SPECIFICATION

(45) Date of publication of patent specification: **19.11.92**  (51) Int. Cl.⁵: **A61M 39/00**, F16L 47/00

(21) Application number: **88903049.0**

(22) Date of filing: **09.03.88**

(86) International application number:
**PCT/US88/00692**

(87) International publication number:
**WO 88/06901 (22.09.88 88/21)**

(54) **MULTIPLE FLUID PATH JUNCTION DEVICE.**

(30) Priority: **12.03.87 US 25139**

(43) Date of publication of application:
**04.07.90 Bulletin 90/27**

(45) Publication of the grant of the patent:
**19.11.92 Bulletin 92/47**

(84) Designated Contracting States:
**AT BE CH DE FR GB IT LI LU NL SE**

(56) References cited:
| | |
| --- | --- |
| FR-A- 2 454 307 | US-A- 1 755 899 |
| US-A- 3 710 942 | US-A- 3 934 902 |
| US-A- 4 497 758 | US-A- 4 566 493 |

(73) Proprietor: **VERNAY LABORATORIES,INC.
P.O. Box 310, 116 South College Street
Yellow Springs, OH 45383(US)**

(72) Inventor: **EDWARDS, Floyd, V.
3325 Fishworm Road
Cedarville, OH 45314(US)**

(74) Representative: **Warren, Keith Stanley et al
BARON & WARREN 18 South End Kensington
London W8 5BU(GB)**

## Description

The present invention relates generally to flow regulation apparatus, and more particularly, to an assembly used in connecting multiple fluid flow paths. Typically, such a device is used to merge multiple inputs to a single output, or to provide means for introducing secondary fluids into a primary fluid flow.

It is common to use fluid flow apparatus in connection with the medical treatment of patients. Fluids such as blood, plasma or medication are often directed into the patient's body, typically through a needle inserted into a vein of a patient.

In many such cases, it is necessary to combine fluids or temporarily replace one fluid with another prior to entry into the patient. For example, a plasma or other fluid may be continuously supplied to the patient. A medicine may be combined with the plasma in a desired ratio, or may simply replace the plasma flow until the proper dosage has been administered. In any case, the combined or replacement flow should continue in an uninterrupted manner, without introducing air or contaminants into the flow. Also, backflow of the secondary fluid into the primary fluid supply must not occur.

For use in such situations, devices have been commercially available for some time which are commonly referred to as injection sites or Y-sites. One such device is disclosed in U.S. Patent 3,710,942 (Rosenburg). Typically, these devices include two or more inlets merging to a single outlet. The primary inlet connects with the primary fluid supply, while the secondary inlet includes means through which the additional substance is introduced. This means can be a rubber plug or other resilient member through which a syringe needle is inserted to inject the fluid into the primary flow. Alternatively, the secondary inlet may include adapter means for connecting the inlet with some other fluid source.

To enable a secondary fluid to be introduced under a higher pressure head than the primary fluid to replace the primary fluid flow, it is necessary to include a check valve in the primary fluid flow path, at or near the primary inlet to the injection site, upstream from the point at which the primary and secondary inlets intersect. As the higher pressure secondary fluid moves into the primary flow path, it will close the check valve, thereby stopping primary fluid flow. Also, in the event of blockage or some other problem in the outlet flow path below the injection site, the check valve operates to prevent the secondary fluid from entering and contaminating the primary fluid supply.

A variety of different check valves may be used for this purpose, with one possibility being a valve commonly known as the "duckbill" type. A typical duckbill valve, and its use within an injection site or Y-site, is shown in US-A-4 535 818. Such a valve typically includes a housing into which is mounted a valving member having as its primary operative components a pair of lips arranged in a converging relationship from an open end to a normally closed end. At the normally closed end of the valving member, the lips are located adjacent each other so as to define a normally closed slit therebetween. The valving member is mounted within the housing so that flow through the housing must pass through the valving member as well. In a first or forward direction, flow enters the housing and passes into the valving member through its open end, moving toward the normally closed end. The flow pressure against the resilient lips opens the slit, allowing the flow to pass out of the valving member and the housing. When flow enters the housing from a second or reverse direction, flow contacts the valving member at its normally closed end, with the flow pressure against the resilient lips holding the slit in its closed position, thereby preventing back flow through the valve assembly.

Due to the complexity of a molded injection site incorporating the housing for the valving member, it is typical to form both the injection site and the check valve housing as separate parts. This is a disadvantage, since further assembly of the parts is required after molding. Moreover, the injection site, even in the absence of the check valve, is a relatively complex part to mold. The formation of a branch conduit requires a side pull mold and tooling. In addition, pins which are used to form the fluid paths must meet at the junction of the fluid paths, thereby producing flashing on the interior of the injection site. Because the flash occurs at the path junction, it is extremely difficult, if not impossible, to remove from the finished molding. Further, in order to permit positioning of plugs or connection adapters within the secondary fluid paths, such paths must be left relatively open with separately molded collars or the like used to secure the additional fittings.

What is needed therefore, is an injection site or other junction device for multiple fluid paths which overcomes the various disadvantages noted above. Such a device should require a simple tooling and molding process to produce, should require little assembly and use a minimum of separate parts. Further, such a device should incorporate or be easily connectable to a check valve. Such a device should reduce flashing in the finished assembly, and should be capable of use with a variety of stoppers, connection adapters or other fittings.

Accordingly, the present invention consists in a multiple fluid path junction device, including first and second housing portions, each of said first and second housing portions defining an inner surface, each of said inner surfaces defining a plurality of intersecting channels extending to the ends of said inner surface, a body defined by joining said first and second housing portions along said inner surfaces thereof, said channels being such that by assembling said first and second housing portions, they cooperate and are aligned to form passageways through said body, a check valve disposed in said body and constructed as a single piece from a resilient material, said valve including a pair of planar lips arranged in a converging relationship to define for said valve an open end and a normally closed end whereat said lips are disposed adjacent each other to define a normally closed slit therebetween, said valve further including at least one side wall interconnecting said lips, a first of said channels in each of said first and second housing portions including, adjacent the end of said inner surface, a widened portion in said housing portion for receiving said valve, each of said widened portions cooperating, upon assembly of said housing portions, to define a containment chamber for said valve, and means for securing said valve within said containment chamber to cause fluid flow into said chamber to pass through said valve, characterized by alignment means formed on said first and second housing portions for aligning said first and second housing portions upon assembly.

The first and second housing portions may be joined by ultrasonic welding.

A second of the channels in each of the first and second housing portions may include, adjacent the end of the inner surface, a second widened portion in the housing portion, this second widened portion including an inwardly extending flange. The junction device then further includes a stopper means fittable within the second widened portions of the first and second housing portions, the stopper means being secured within the body by the flange.

The alignment means may include a flange extending along one edge of each of the first and second housing portions, the flange engaging an opposite edge on the opposite of the housing portions upon joining of the housing portions. The alignment means may also include a pin defined in the first housing portion and an opening defined in the second housing portion, the pin engaging the opening upon joining of the housing portions.

The first and second housing portions are preferably identical.

One of the channels in each housing portion may form a primary passageway, with the widened portion for receiving the valve being disposed along the primary passageway. Another of the channels in each housing portion may form a secondary passageway, with the secondary passageway extending from the primary passageway at an acute angle with respect to the widened portion.

An additional channel in each housing portion may form a further secondary passageway extending from the primary passageway, both of the secondary passageways extending symmetrically with respect to the primary passageway.

The present invention also includes a method of constructing a multiple fluid path junction device, including a check valve, comprising the steps of molding first and second housing portions such that each of said first and second portions defines an inner surface, with each of said inner surfaces defining a plurality of intersecting channels extending to the ends of said inner surface, positioning said first and second housing portions together to form a body by placing said inner surfaces in contact with each other, whereby said channels align to form passageways through said body, securing said housing portions to hold said housing portions together, said molding of said first and second housing portions being further performed such that a first of said channels in each of said first and second housing portions is formed to include, adjacent the end of said inner surface, a widened portion, within said housing portion, which defines a containment chamber, placing into said containment chamber a check valve constructed as a single piece from a resilient material, said valve including a pair of planar lips arranged in a converging relationship to define for said valve an open end and a normally closed end whereat said lips are disposed adjacent each other to define a normally closed slit therebetween, said valve further including at least one side wall interconnecting said lips, and securing said housing portions together to secure said valve within said containment chamber to cause fluid flow into said chamber to pass through said valve, characterized by ensuring proper alignment of said housing portions together by effecting the mating engagement of alignment means which are provided in said first and second portions.

The housing portions may be molded from a thermoplastic material. Also, the housing portions may be secured by ultrasonic welding.

In order that the invention may be more readily understood, reference will now be made to the accompanying drawings, in which:

Fig. 1 illustrates a multiple fluid path junction device in accordance with the present invention, used in conjunction with a medical fluid administration apparatus;

Fig. 2 is a sectional view of the multiple fluid path junction device;

Fig. 3 is an end view of the device with cap, valve and stoppers removed; and

Fig. 4 is a sectional view of the check valve portion of the device, taken generally along line 4--4 of Fig. 2.

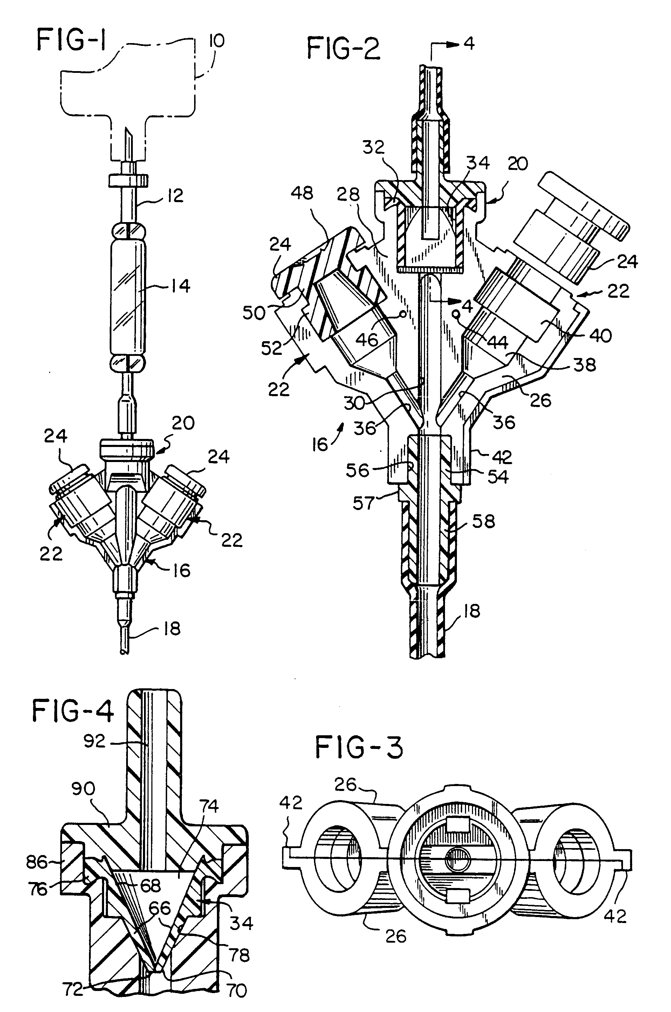

A fluid path junction device in accordance with the present invention may be seen by reference to Fig. 1. The device is shown in a typical environment, specifically as part of a medical solution and administration set. Such a set is used to deliver fluids, such as plasma, medicines, and the like from a fluid supply source 10, typically a bottle or bag, intravenously to a patient. Supply 10 is coupled by connector 12 to a drip chamber 14. Fluid then passes into the multiple path junction device 16, from which the fluid emerges into tubing 18 to direct it to the lower end of the fluid path for connection to a needle (not shown) which is inserted into the vein of a patient.

Of course, the environment shown in Fig. 1 is presented by way of example. Many other uses for the junction device shown herein will be readily apparent to those skilled in the art.

Junction device 16 and its construction includes an inlet portion 20 having a check valve located therein in a manner to be described in detail below. A pair of side or secondary inlets 22 connect with the primary through device 16, with each inlet 22 being closed by a rubber stopper 24 or other appropriate device. When used with the solution set shown in Fig. 1, medicines or other materials can be directed into the primary fluid stream by inserting a needle through stopper 24 and injecting the substance through the needle. The injected substance then replaces or combines with the primary fluid flow through device 16, and is conveyed to the patient.

Junction device 16 and its construction can be seen in detail by reference to Figs. 2 and 3. The major portion of device 16 is formed from a pair of body halves 26 which are identical in shape and are assembled together to define a housing device 16. Halves 26 are molded parts, and may be formed from acrylic, ABS, polycarbonate or other rigid plastic. As seen in Fig. 2, each half 26 defines an inner surface 28 which has formed therein in a primary channel 30 extending substantially the full length of half 26. Near inlet end 20, channel 30 opens into a valve housing portion 32, within which a duckbill type check valve regulator 34 is located. Housing portion 32 and regulator 34 will be described in detail below.

A pair of side channel 36 branches from primary channel 30. Each side channel 36 opens into a semi-circular inlet chamber 38, which includes an expanded portion 40 having a greater radius than the remaining portion of chamber 38. Each inlet chamber 38 opens along the edge of half 26 to define one inlet 22.

To form the body of the junction device 16, two halves 26 are placed with their respective inner surfaces 28 located in contact. To aid in properly positioning the two halves 26, a lip or ridge 42 is provided along one side edge of each half 26. As can be best seen by Fig. 3, lip 42 on each edge cooperates to properly locate the two halves once placed together, and to guide the joining of halves 26, as well as to hide the joint therebetween. Additionally, a pin 44 extending upwardly from surface 28 cooperates with an opening 46 located on the surface 28 of the opposite half 26. Once positioned, the two halves 26 are secured together, preferably by ultrasonic welding, but alternatively by gluing or the like.

It can be seen that after assembly, the respective primary channels 30 of each half 26 together define a primary passageway which is circular in cross-section. Additionally, side channels 36 cooperate to define side passageways which are circular in cross-section, as are the side inlet chambers 38.

Prior to assembly, stoppers 24 are fitted into portions 38 of each side path. As can be seen in Fig. 2, stopper 24 includes a plug portion 48, a neck portion 50, and shoulder portion 52. Shoulder portion 52 is placed within widened expanded portion 40 of recess 38, so that upon connecting halves 26, stopper 24 is firmly secured within the secondary inlet of device 16. It should be noted that the shoulder portion 52 enables the stoppers to be firmly held without the use of additional collars or other parts, and without separately gluing the stoppers in place.

An outlet connector 54 is located within an outlet recess 56 provided at one end of primary channel 30. Outlet connector 54 includes a positioning flange 57 and is welded in position at the same time halves 26 are joined together. Connector 54 provides a connection portion 58 for joining with outlet tubing 18 when device 16 is placed into use.

Flow regulator 34, which defines the operating portion of the check valve located at the inlet end of device 16, can be seen in greater detail by reference to Fig. 4. Regulator member 34, molded as a single piece from a material such as rubber or resilient plastic, is formed in a roughly conical, hollow shape. A pair of lips 66 form a portion of regulator 34, each lip 66 having a base region 68 and an outer end 70. Lips 66 are disposed in a converging relationship so that lips 66 are adjacent each other at their outer end 70. Lips 66 thus form a normally closed slit 72 to define a normally closed end for regulator 34.

Curved side walls 74 interconnect lips 66 and define an open end for regulator 34 opposite slit 72. An outwardly extending flange 76 is formed around the open end of regulator 34 adjacent the bases 68 of lips 66.

Portions 32 of halves 26 together define a complete valve housing having an interior that approximates the shape of regulator 34. A pair of planar walls 78 define a pair of planar surfaces for the housing interior. Planar walls 78 are arranged in a converging relationship toward the entry to the primary passageway defined by channels 30.

Planar walls 78 are connected by curved walls, and an annular collar 86 extends around the open end of the outlet portion. As a result, an annular shelf is defined, so that regulator 34 may be positioned within the housing interior by placement of flange 76 against collar 86. When so placed, lips 66 are disposed substantially adjacent to, but not in contact with, planar walls 78. Outer ends 70 of lips 66 are then located at the entry wall to the primary passage, so that fluid flow passing through slit 72 is directed into the central passage.

A circular cover plate 90 is also provided, through which an inlet port 92 communicates. After regulator 34 is placed within the housing interior, cover plate 90 is positioned so that cover plate 90 is in contact with annular collar 76 on regulator 34, thereby confining flange 76 and providing a sealed housing for regulator 34.

Further details regarding the check valve construction and its operation may be seen by reference to US-A-4 612 960.

In the embodiment disclosed herein, the side paths of the junction device include the stoppers shown and described. However, it should be recognized that connections for luer-locks or other devices can be substituted for the stoppers to provide a junction device capable of other uses. Similarly, check valves similar to that disposed in the primary fluid passageway may be located in the secondary passageways to provide a three-way check valve manifold. Or the device could be formed as a simple manifold device without any check valves.

It should also be recognized that it is not necessary that three fluid inlets be provided for the device. For example, two inlets could be provided with the device assuming a Y-shaped configuration. Alternatively, the primary pathway could be maintained straight with a single side path branching therefrom. However, in this latter case the resulting halves of the device will not be identical. This will require two separate molds with the resulting parts requiring separate handing prior to assembly to form the completed device.

It should further be noted that the volume of retained fluid within the device is very small due to the close conformance of the flow paths. Further, no sharp corners are directly exposed to the fluid flow, which could otherwise introduce turbulence and decrease flow in the fluid. Moreover, because a side pull technique is not used for molding the device, flashing at the path junctions is also eliminated.

## Claims

1. A multiple fluid path junction device, including:

   first and second housing portions (26), each of said first and second housing portions defining an inner surface, each of said inner surfaces defining a plurality of intersecting channels (30, 36) extending to the ends of said inner surface:

   a body defined by joining said first and second housing portions along said inner surfaces thereof;

   said channels being such that by assembling said first and second housing portions, they cooperate and are aligned to form passageways through said body;

   a check valve (34) disposed in said body and constructed as a single piece from a resilient material, said valve including a pair of planar lips (66) arranged in a converging relationship to define for said valve an open end and a normally closed end whereat said lips are disposed adjacent each other to define a normally closed slit (72) therebetween, said valve further including at least one side wall (74) interconnecting said lips;

   a first of said channels (30) in each of said first and second housing portions including, adjacent the end of said inner surface, a widened portion (32) in said housing portion for receiving said valve (34), each of said widened portions cooperating, upon assembly of said housing portions, to define a containment chamber for said valve; and

   means (90) for securing said valve within said containment chamber to cause fluid flow into said chamber to pass through said valve,

   characterized by:

   alignment means (42, 44, 46) formed on said first and second housing portions for aligning said first and second housing portions upon assembly.

2. The device as defined in claim 1, wherein said first and second housing portions (20) are joined by ultra-sonic welding.

3. The device as defined in claim 1 or 2, wherein a second of said channels (36) in each of said first and second housing portions includes, adjacent the end of said inner surface, a second widened portion (40) in said housing portion, said second widened portion including an inwardly extending flange therein, and wherein said device further comprises a stopper means (24) fittable within said second widened portions of said first and second housing portions, said stopper means being secured within said body by said flange.

4. The device as defined in claim 1, 2 or 3 wherein said alignment means includes a flange (42) extending along one edge of each of said first and second housing portions, said flange engaging an opposite edge on the opposite of said housing portions upon joining of said housing portions.

5. The device as defined in claim 1, 2, 3 or 4, wherein said alignment means includes a pin (44) defined in said first housing portion and an opening (46) defined in said second housing portion, said pin engaging said opening upon joining of said housing portions.

6. The device as defined in any preceding claim, wherein said first and second housing portions (20) are identical.

7. The device as defined in any preceding claim, wherein one of said channels in each housing portion forms a primary passageway (30), said widened portion for receiving said valve being disposed along said primary passageway, and wherein another of said channels in each housing potion forms a secondary passageway (36), said secondary passageway extending from said primary passageway at an acute angle with respect to said widened portion.

8. The device as defined in claim 7, wherein an additional one of said channels in each housing portion forms a further secondary passageway (36) extending from said primary passageway, both of said secondary passageways extending symmetrically with respect to said primary passageway.

9. The device as defined in any preceding claim, wherein said securing means includes an end cap (90) having an inlet port (92) defined therethrough, said cap being secured along an edge of said body over said containment chamber and said valve positioned therein.

10. A method of constructing a multiple fluid path junction device, including a check valve, comprising the steps of:
   molding first and second housing portions (20) such that each of said first and second portions define an inner surface, with each of said inner surfaces defining a plurality of intersecting channels (30, 36) extending to the ends of said inner surface;
   positioning said first and second housing portions together to form a body by placing said inner surfaces in contact with each other, whereby said channels align to form passageways through said body;
   securing said housing portions to hold said housing portions together;
   said molding of said first and second housing portions being further performed such that a first of said channels in each of said first and second housing portions is formed to include, adjacent the end of said inner surface, a widened portion (32), within said housing portion, which defines a containment chamber;
   placing into said containment chamber a check valve (34) constructed as a single piece from a resilient material, said valve including a pair of planar lips (66) arranged in a converging relationship to define for said valve an open end and a normally closed end whereat said lips are disposed adjacent each other to define a normally closed slit (72) therebetween, said valve further including at least one side wall (74) interconnecting said lips;
   and securing said housing portions together to secure said valve within said containment chamber to cause fluid flow into said chamber to pass through said valve;
   characterized by:
   ensuring proper alignment of said housing portions together by effecting the mating engagement of alignment means (42, 44, 46) which are provided in said first and second portions.

11. The method as defined in claim 10, wherein said housing portions are molded from a thermoplastic material.

12. The method as defined in claim 11, wherein said housing portions are secured by ultrasonic welding.

**Patentansprüche**

1. Mehrwegeverbindungsvorrichtung für Fließmittel mit:
   ersten und zweiten Gehäuseteilen (26), deren jedes eine Oberfläche bildet, wobei jede der inneren Oberflächen eine Mehrzahl von sich schneidenden Kanälen (30,36) bildet, die sich zu den Enden der inneren Oberfläche erstrecken;
   einem Körper, welcher durch Verbinden der ersten und zweiten Gehäuseteile längs ihrer inneren Oberflächen gebildet ist;
   wobei die Kanäle derart sind, daß sie durch Anordnen der ersten und zweiten Gehäuseteile zusammenwirken und ausgerichtet sind, um Durchgänge durch den Körper zu bilden;

einem Absperrventil (34), das in dem Körper angeordnet und als Einzelstück aus einem federnd elastischen Material aufgebaut ist, wobei das Ventil ein Paar von ebenen Lippen (66) aufweist, die konvergierend angeordnet sind, um für das Ventil ein offenes Ende und ein normalerweise geschlossenes Ende zu bilden, während die Lippen nebeneinander angeordnet sind, um dazwischen einen normalerweise geschlossenen Schlitz (72) zu bilden, wobei das Ventil ferner mindestens eine die Lippen verbindende Seitenwand (74) aufweist;

wobei ein erster der Kanäle (30) in jedem der ersten und zweiten Gehäuseteile neben dem Ende der inneren Oberfläche einen aufgeweiteten Teil (32) in dem Gehäuse aufweist für die Aufnahme des Ventils (34), wobei jeder der aufgeweiteten Teile nach Zusammenbau der Gehäuseteile zusammenwirkt, um eine Einschließungskammer für das Ventil zu bilden; und

Mitteln (90) für die Befestigung des Ventils in der Einschließungskammer, um das Fließmittel zu veranlassen, in die Kammer hineinzuströmen und durch das Ventil hindurchzugehen,

gekennzeichnet durch

Ausrichtmittel (42, 44, 46), die auf den ersten und zweiten Gehäuseteilen gebildet sind zum Ausrichten der ersten und zweiten Gehäuseteile auf den Zusammenbau hin.

2. Vorrichtung nach Anspruch 1, wobei die ersten und zweiten Gehäuseteile (20) durch Ultraschallschweißen verbunden sind.

3. Vorrichtung nach Anspruch 1 oder 2, wobei ein zweiter der Kanäle (36) in jedem der ersten und zweiten Gehäuseteile nahe dem Ende der inneren Oberfläche einen zweiten aufgeweiteten Teil (40) in dem Gehäuseteil aufweist, wobei der zweite aufgeweitete Teil einen sich nach einwärts erstreckenden Flansch in diesem aufweist und wobei die Vorrichtung ferner ein Anschlagmittel (24) aufweist, das innerhalb des zweiten aufgeweiteten Teils der ersten und zweiten Gehäuseteile einpaßbar ist, wobei das Anschlagmittel in dem Körper durch den Flansch befestigt ist.

4. Vorrichtung nach Anspruch 1, 2 oder 3, wobei das Ausrichtmittel einen Flansch (42) aufweist, der sich längs einer Kante jedes der ersten und zweiten Gehäuseteile erstreckt, wobei der Flansch mit einer gegenüberliegenden Kante auf dem gegenüberliegenden Gehäuseteil auf das Verbinden der Gehäuseteile in Eingriff tritt.

5. Vorrichtung nach Anspruch 1, 2, 3 oder 4, wobei das Ausrichtmittel einen Stift (44) aufweist, der in dem ersten Gehäuseteil gebildet ist, und eine Öffnung (46) aufweist, die in dem zweiten Gehäuseteil gebildet ist, wobei der Stift auf das Verbinden der Gehäuseteile hin mit der Öffnung in Eingriff tritt.

6. Vorrichtung nach einem vorhergehenden Anspruch, wobei die ersten und zweiten Gehäuseteile (20) identisch sind.

7. Vorrichtung nach einem vorhergehenden Anspruch, wobei einer der Kanäle in jedem Gehäuseteil einen primären Durchgang (30) bildet, der aufgeweitete Teil für die Aufnahme des Ventils längs des primären Durchganges angeordnet ist und wobei ein anderer der Kanäle in jedem Gehäuseteil einen zweiten Durchgang (36) bildet, der sich von dem ersten Durchgang in einem spitzen Winkel bezüglich des aufgeweiteten Teiles erstreckt.

8. Vorrichtung nach Anspruch 7, wobei ein zusätzlicher Kanal in jedem Gehäuseteil einen weiteren zweiten Durchgang (36) bildet, der sich von dem primären Durchgang erstreckt, wobei beide zweite Durchgänge symmetrisch bezüglich des primären Durchganges verlaufen.

9. Vorrichtung nach einem vorhergehenden Anspruch, wobei das Befestigungsmittel eine Endkappe (90) aufweist, in welcher eine durchgehende Einlaßöffnung (92) angeordnet ist, wobei die Kappe entlang einer Kante des Körpers über der Einschließungskammer angebracht ist und das Ventil darin angeordnet ist.

10. Verfahren zum Aufbau einer Mehrwegeverbindungsvorrichtung für Fließmittel, mit einem Absperrventil, mit folgenden Schritten:

Formen erster und zweiter Gehäuseteile (20) derart, daß jedes der ersten und zweiten Gehäuseteile eine innere Oberfläche bildet, wobei jede der inneren Oberflächen eine Mehrzahl von sich schneidenden Kanälen (30, 36) bildet, die sich zu den Enden der inneren Oberfläche erstrecken;

Zusammenanordnen der ersten und zweiten Gehäuseteile, um einen Körper zu bilden durch Anordnen der inneren Oberflächen in Berührung miteinander, wodurch die Kanäle in Flucht kommen, um Durchgänge durch den Körper hindurch zu bilden;

Befestigen der Gehäuseteile, um die Gehäuseteile zusammenzuhalten;

wobei das Formen der ersten und zweiten Gehäuseteile ferner derart durchgeführt wird, daß ein erster der Kanäle in jedem der ersten und zweiten Gehäuseteile gebildet ist, um in der Nähe des Endes der inneren Oberfläche einen aufgeweiteten Teil (32) in dem Gehäuseteil einzuschließen, weicher eine Einschließungskammer bildet;

Anordnen eines Absperrventiles (34) in die Einschließungskammer hinein, wobei das Absperrventil als Einzelstück aus einem elastisch federnden Material aufgebaut ist und ein Paar von ebenen Lippen (66) aufweist, die konvergierend angeordnet sind, um für das Ventil ein offenes Ende und ein normalerweise geschlossenes Ende zu bilden, wobei die Lippen nebeneinander angeordnet sind, um dazwischen einen normalerweise geschlossenen Schlitz (72) zu bilden, wobei das Ventil ferner mindestens eine die Lippen schneidende Seitenwand (74) aufweist;

und Befestigen der Gehäuseteile aneinander, um das Ventil in der Einschließungskammer zu sichern und das Fließmittel zu veranlassen, in die Kammer hineinzufließen und durch das Ventil zu gehen;

gekennzeichnet durch:

Sicherstellen eines guten Zusammenausrichtens der Gehäuseteile durch den passenden Eingriff der Ausrichtmittel (42, 44, 46), die in den ersten und zweiten Teilen vorgesehen sind.

11. Verfahren nach Anspruch 10, wobei die Gehäuseteile aus einem thermoplastischen Material geformt werden.

12. Verfahren nach Anspruch 11, wobei die Gehäuseteile durch Ultraschallschweißen befestigt werden.

**Revendications**

1. Dispositif destiné à joindre plusieurs trajets fluides, qui comprend :

une première et une deuxième portions de carter (26), chacune desdites première et deuxième portions de carter définissant une surface interne, chacune desdites surfaces internes définissant une pluralité de canaux (30, 36) qui se coupent et s'étendent vers les extrémités de ladite surface interne ;

un corps défini par la réunion desdites première et deuxième portions de carter le long desdites surfaces intérieures de ces portions ;

lesdits canaux étant tels que, après assemblage desdites première et deuxième portions de carter, ils coopèrent et sont alignés pour former des passages à travers ledit corps ;

un clapet de retenue (34), disposé dans ledit corps et construit d'une manière monobloc en un matériau résilient, ledit clapet comprenant une paire de lèvres planes (66) disposées selon une relation de convergence de façon à définir pour ledit clapet une extrémité ouverte et une extrémité normalement fermée, lesdites lèvres étant disposées d'une manière contiguë l'une par rapport à l'autre pour définir entre elles une fente (72) normalement fermée, ledit clapet comprenant en outre au moins une paroi latérale (74) qui relie lesdites lèvres l'une à l'autre ;

un premier desdits canaux (30), dans chacune desdites première et deuxième portions de carter, définissant, en un point situé au voisinage immédiat de l'extrémité de ladite surface interne, une portion élargie (32) dans ladite portion de carter, destinée à recevoir ledit clapet (34), chacune desdites portions élargies coopérant, après assemblage desdites portions de carter, pour définir une chambre de confinement destinée audit clapet ; et

un moyen (90) destiné à fixer ledit clapet dans ladite chambre de confinement, de façon à faire s'écouler un fluide dans ladite chambre pour qu'il traverse ledit clapet,

caractérisé par des moyens d'alignement (42, 44, 46), formés sur lesdites première et deuxième portions de carter, et servant à aligner lesdites première et deuxième portions de carter après assemblage.

2. Dispositif selon la revendication 1, dans lequel lesdites première et deuxième portions de carter (20) sont assemblées par un soudage aux ultrasons.

3. Dispositif selon la revendication 1 ou 2, dans lequel un deuxième desdits canaux (36), dans chacune desdites première et deuxième portions de carter, comprend, au voisinage immédiat de l'extrémité de ladite surface interne, une deuxième portion élargie (40) dans ladite portion de carter, ladite deuxième portion élargie comprenant une bride s'étendant vers l'intérieur, et où ledit dispositif comprend en outre un moyen d'obturation (24), pouvant être inséré dans lesdites deuxièmes portions élargies desdites première et deuxième portions de carter, ledit moyen d'obturation étant fixé dans ledit corps au moyen de ladite bride.

4. Dispositif selon la revendication 1, 2 ou 3, dans lequel ledit moyen d'alignement comprend une bride (42) qui s'étend le long d'une extrémité de chacune desdites première et deuxième portions de carter,ladite bride entrant en prise avec un bord opposé, sur le côté opposé desdites portions de carter, après assemblage desdites portions de carter.

5. Dispositif selon la revendication 1, 2, 3 ou 4, dans lequel ledit moyen d'alignement comprend une cheville (44) définie dans ladite première portion de carter et une ouverture (46) définie dans ladite deuxième portion de carter, ladite cheville entrant dans ladite ouverture après assemblage desdites portions de carter.

6. Dispositif selon l'une quelconque des revendications précédentes, dans lequel ladite première portion de carter et ladite deuxième portion de carter (20) sont identiques.

7. Dispositif selon l'une quelconque des revendications précédentes, dans lequel l'un desdits canaux se trouvant dans chaque portion de carter forme un passage primaire (30), ladite portion élargie, destinée à recevoir ledit clapet, étant disposée le long dudit passage primaire, et où un autre desdits canaux se trouvant dans chaque portion de carter forme un passage secondaire (36), ledit passage secondaire s'étendant à partir dudit passage primaire, en faisant un angle aigu avec ladite portion élargie.

8. Dispositif selon la revendication 7, dans lequel un canal supplémentaire desdits canaux se trouvant dans chaque portion de carter forme un autre passage secondaire (36) s'étendant à partir dudit passage primaire, ces deux passages secondaires s'étendant symétriquement par rapport audit passage primaire.

9. Dispositif selon l'une quelconque des revendications précédentes, dans lequel ledit moyen de fixation comprend un chapeau en bout (90) comportant un orifice d'entrée (92) défini à travers lui, ledit chapeau étant fixé le long d'un bord dudit corps, sur ladite chambre de confinement, ledit clapet y étant placé.

10. Procédé de construction d'un dispositif pour joindre plusieurs trajets fluides, comprenant un clapet de retenue, et qui comprend les étapes consistant :

à mouler une première et deuxième portions de carter (20), de façon que chacune desdites première et deuxième portions définissent une surface interne, chacune desdites surfaces internes définissant une pluralité de canaux (30, 36) qui se coupent et s'étendent jusqu'aux extrémités de ladite surface interne ;

a positionner lesdites première et deuxième portions de carter l'une avec l'autre de façon à former un corps, en mettant en contact l'une avec l'autre lesdites surfaces internes, lesdits canaux s'alignant pour former des passages à travers ledit corps ;

à fixer lesdites portions de carter de façon à maintenir ensemble lesdites portions de carter ;

ledit moulage desdites première et deuxième portions de carter étant de plus mis en oeuvre du façon qu'il se forme un premier desdits canaux dans chacun desdites première et deuxième portions de carter, et qui comprenne, au voisinage immédiat de l'extrémité de ladite surface interne, une portion élargie (32), à l'intérieur de ladite portion de carter, qui définit une chambre de confinement ;

à placer dans ladite chambre de confinement un clapet de retenue (34) réalisé d'une manière monobloc à partir d'un matériau résiliant, ledit clapet comprenant une paire de lèvres planes (66) disposées selon une relation de convergence de façon à définir pour ledit clapet une extrémité ouverte et une extrémité normalement fermée, lesdites lèvres étant disposées au voisinage immédiat l'une de l'autre de façon à définir entre elles une fente normalement fermée (72), ledit clapet comprenant en outre au moins une paroi latérale (74) qui relie lesdites lèvres ;

et à fixer l'une à l'autre lesdites portions de carter pour fixer ledit clapet à l'intérieur de ladite chambre de confinement de façon qu'un fluide puisse s'écouler dans ladite chambre pour traverser ledit clapet ;

caractérisé en ce qu'il consiste à garantir un alignement approprié desdites portions de carter l'une par rapport à l'autre, grâce à un contact par appariement des moyens d'alignement (42, 44, 46), qui sont prévus dans lesdites première et deuxième portions.

11. Procédé selon la revendication 10, dans lequel lesdites portions de carter sont moulées à partir d'un matériau thermoplastique.

12. Procédé selon la revendication 11, dans lequel lesdites portions de carter sont fixées par soudage aux ultrasons.

EP 0 375 681 B1

FIG-1

FIG-2

FIG-4

FIG-3

10